(19) Europäisches Patentamt
European Patent Office
Office européen des brevets

(11) Veröffentlichungsnummer : **0 081 691**
**B1**

(12) **EUROPÄISCHE PATENTSCHRIFT**

(45) Veröffentlichungstag der Patentschrift : **13.03.85**

(51) Int. Cl.⁴ : **C 07 C 101/12**, C 11 D 1/90

(21) Anmeldenummer : **82110486.6**

(22) Anmeldetag : **13.11.82**

(54) **Bis-Betaine, Verfahren zu deren Herstellung und diese enthaltende Reinigungsmittel.**

(30) Priorität : **19.11.81 DE 3145733**

(43) Veröffentlichungstag der Anmeldung : **22.06.83 Patentblatt 83/25**

(45) Bekanntmachung des Hinweises auf die Patenterteilung : **13.03.85 Patentblatt 85/11**

(84) Benannte Vertragsstaaten : **AT BE CH DE FR GB IT LI NL SE**

(56) Entgegenhaltungen : **US-A- 3 265 719**

(73) Patentinhaber : **HOECHST AKTIENGESELLSCHAFT**
**Postfach 80 03 20**
**D-6230 Frankfurt am Main 80 (DE)**

(72) Erfinder : **Blaschke, Günter, Dr.**
**Bajuwarenstrasse 36**
**D-8261 Winhöring (DE)**
Erfinder : **Reng, Alwin**
**Im Schulzehnten 22**
**D-6233 Kelkheim (Taunus) (DE)**
Erfinder : **Quack, Jochen Meinhard, Dr.**
**Wilhelm-Reuter-Strasse 16**
**D-6239 Eppstein/Taunus (DE)**

**EP 0 081 691 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Zur Entfernung von exogenen und endogenen Verunreinigungen von Haut und Haaren werden üblicherweise anionische, kationische, amphotere oder nicht-ionische Verbindungen allein oder in Form von Kombinationen innerhalb dieser Gruppen verwendet. Die Mehrzahl der auf dem Markt befindlichen Körper- und Haarreinigungsmittel werden mit anionischen Tensiden vom Typ Seifen, Alkylsulfate, Alkylethersulfate, Sulfobernsteinsäureester, sekundäre Alkylsulfonate, α-Olefinsulfonate, Acylaminopolyglykolethersulfate oder Sarkoside hergestellt. Diese Substanzen weisen bei der Anwendung in der Praxis eine Reihe von Nachteilen auf. Beispielsweise werden die Haare häufig zu stark entfettet, so daß hierdurch die Kämmbarkeit der nassen und der trockenen Haare erheblich verschlechtert wird, außerdem wird der Griff der Haare negativ beeinträchtigt. Bei dem Einsatz der üblichen Tenside in Körperreinigungsmitteln, wie beispielsweise Handwaschmitteln oder Duschbädern, verursacht die zu starke Reinigungswirkung eine trockene und gespannte Haut. Gleichzeitig wird durch die Adsorption der anionischen Tenside ein unangenehmes « klebriges » Hautgefühl nach der Anwendung beobachtet. Es wurde schon versucht, durch Zusatz von kationischen Tensiden diese Nachteile zu umgehen, wobei es jedoch in den Formulierungen durch die Bildung von Elektroneutralsalzen zwischen anionischen und kationischen Tensiden oft zu Trübungen oder Ausfällungen kommt. Auch der Zusatz von polymeren, kationischen Substanzen verursacht oft Schwierigkeiten durch Reduzierung der Schaumbildung und zu hohe Adsorption auf den Haaren, die zu einem unangenehmen Akkumulationseffekt führt.

Auch amphotere Tenside mit einer Betain-Gruppe im Molekül sind versuchsweise allein oder in Kombination mit anionischen Tensiden benutzt worden.

Aus der DE-AS 12 49 433 ist beispielsweise der Einsatz von Alkyl-Betainen in Reinigungsmitteln bekannt, während Amidoalkyl-Betaine der Formel $R^1CONH \cdot (CH_2)_x \cdot N^+R^2R^3 \cdot (CH_2)_y \cdot COO^-$, worin $R^1$ der Alkylrest einer Fettsäure ist, in der DE-AS 11 72 802 als hautverträgliche Badezusätze, in der DE-AS 10 62 392 als keimtötende, die Augen nicht reizende Haarwaschmittel empfohlen worden sind. Jedoch ist die tensidische Wirkung dieser Mono-Betaine nicht ausreichend. Außerdem besitzen beispielsweise die Alkyl-Betaine eine schlechte Augenschleimhaut-Verträglichkeit und auch diejenige der Amidoalkyl-Betaine ist noch nicht optimal.

Aufgabe der Erfindung ist es daher, neue und verbesserte, die Haut und Augenschleimhaut nicht irritierende Betaine, ein Verfahren zu ihrer Herstellung sowie Haarshampoos und kosmetische Reinigungsmittel, die diese Betaine enthalten, zur Verfügung zu stellen. Insbesondere sollen solche Betaine unter Beibehaltung der übrigen vorteilhaften Eigenschaften bei der Anwendung in Haar- oder Körperreinigungsmitteln einen vom Verbraucher gewünschten Avivage-Effekt bewirken.

Zur Lösung dieser Aufgabe werden erfindungsgemäß Bis-Betaine der allgemeinen Formel

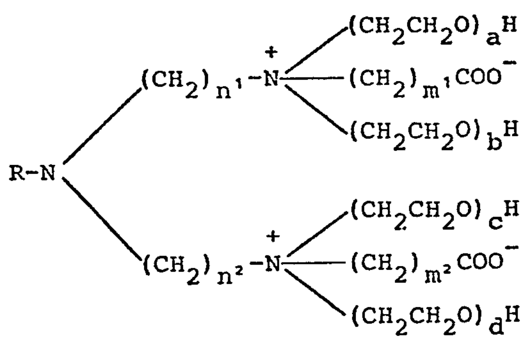 (I)

zur Verfügung gestellt, worin

R einen gesättigten oder einen olefinisch ungesättigten Kohlenwasserstoffrest mit 1 bis 3 Doppelbindungen und von 8 bis 22 C-Atomen bedeutet,

$n^1$ und $n^2$ eine ganze Zahl von 2 bis 3, wobei $n^1$ und $n^2$ gleich oder verschieden sein können,

$m^1$ und $m^2$ eine ganze Zahl von 1 bis 4, wobei $m^1$ und $m^2$ gleich oder verschieden sein können, darstellt, sowie

a, b, c und d, gleich oder verschieden, jeweils eine Zahl von 1 bis 5 ist, wobei die Summe a + b + c + d höchstens 10 betragen soll.

In diesen erfindungsgemäßen Bis-Betainen der Formel I besitzt der Rest R 8 bis 22 C-Atome, er kann gesättigt oder ungesättigt mit 1 bis 3 olefinischen Doppelbindungen und er kann geradkettig oder verzweigt sein. Diese Alkyl- oder Alkenylreste, die dem primären Ausgangsamin bei der Herstellung der erfindungsgemäßen Bis-Betaine entstammen, sind häufig Gemische oder Kettenschnitte, bevorzugt mit der Kettenverteilung der Reste von natürlichen Fettsäuren, wie insbesondere der Kokos-, Talg- oder Palmkernfettsäure, aus denen diese Ausgangsamine über den Weg der Nitrilhydrierung oder der Ammonolyse der entsprechenden Alkohole gewonnen werden können. Die zur Herstellung der primären Amine mittels Ammonolyse verwendeten Alkohole können neben Fettalkoholen auch solche mit gerader

oder verzweigter Kette aus dem Ziegler-Prozeß (Ethylen-Aufbaualkohole) oder aus der Oxosynthese sein.

Zur Herstellung der erfindungsgemäßen Verbindungen wird zunächst ein solches primäres Amin der Formel $RNH_2$, wobei R die vorgenannte Bedeutung hat, mit 2 mol eines reaktionsfähigen Nitrils von 2 bis 3 C-Atomen (einschließlich der CN-Gruppe) oder Gemischen solcher Nitrile zu einer Verbindung der allgemeinen Formel

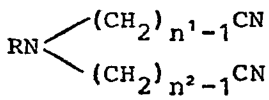

(III)

in einer Dicyanalkylierungsreaktion umgesetzt. Diese Reaktion ist bekannt, beispielsweise aus der US-PS 3 028 415. Sie kann sowohl mit saurer als auch mit basischer Katalyse, mit Hilfe von Lösungsmitteln, wie Wasser oder auch niederkettigen Alkoholen, drucklos oder unter erhöhtem Druck, kontinuierlich oder diskontinuierlich durchgeführt werden. Als saure Katalysatoren werden Essigsäure, Phosphorsäure, Salzsäure und andere Mineralsäuren genannt (US-PS 3 615 797, US-PS 3 028 415, DE-OS 1 941 913), als basische Katalysatoren sind empfohlen worden Natrium- oder Kaliumhydroxid, Alkalialkoholate, Trimethylbenzylammoniumhydroxid und Morpholin (Kirk-Othmer, Encyclopedia of Chemical Technology, 1955, Band 6, Seite 634 ff., H. A. Bruson « Cyanoethylation », Organic Reactions, 5, 1949, Seite 79 ff., Verlag John Wiley and Sons, New York). Als Co-Katalysatoren oder auch als Lösungsvermittler werden Wasser oder niedere Alkohole, wie Methanol, Ethanol, Isopropanol oder Gemische derselben in Anteilen von 1 bis 20 Gew.-% zugegeben. Die Dicyanalkylierung wird unter Normaldruck oder leichtem bis mittlerem Überdruck von 1 bis 20 bar, gegebenenfalls in Gegenwart eines Inertgases, und bei Temperaturen von 60 bis 150 °C durchgeführt. Das Cyanalkylierungsmittel, vorzugsweise Acrylnitril oder Chloracetonitril, wird stöchiometrisch oder in einem bis zu vierfachen Überschuß angewandt.

Anschließend wird das so gewonnene Dicyanalkylierungsprodukt (III) in Gegenwart von Wasserstoff zu einer Verbindung der Formel

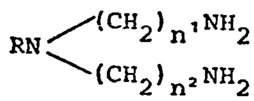

(IV)

reduziert und anschließend mit Ethylenoxid zu einer Verbindung der Formel

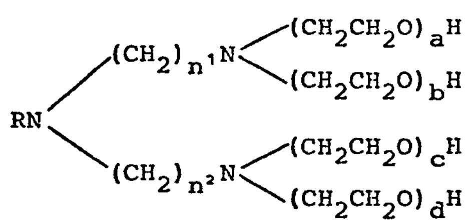

(V)

kondensiert. Beide Reaktionen sind für die in Rede stehenden Verbindungen ebenfalls bekannt (vgl. die oben bereits genannte US-PS 3 615 797). Die Reduktion wird mit Raney-Nickel oder Raney-Kobalt oder aber mit Nickel- oder Kobalt-Trägerkatalysatoren durchgeführt, und zwar unter Einsatz von 1 bis 10 Gew.-% Katalysator, bevorzugt 1 bis 5 Gew.-%, und bei Drücken von 50 bis 200 bar Wasserstoff und Temperaturen von 60 bis 150 °C ; die Reaktionszeit beträgt dabei etwa 1 bis 5 Stunden.

Die Oxethylierungsreaktion wird in Druckgefäßen durchgeführt, und zwar bei erhöhter Temperatur im Bereich von 110 bis 170 °C und erhöhtem Druck von 1 bis 5 bar. Ein Katalysator ist nicht erforderlich, wenn vorzugsweise nur eine Ethylenoxideinheit pro Kette angelagert werden soll. Wird ein Katalysator eingesetzt, so ergeben sich vorzugsweise Ethylenoxidketten, die mehr als eine Einheit enthalten. Es werden 4 bis 10 mol Ethylenoxid pro 1 mol der Verbindung IV in der Reaktion eingesetzt, vorzugsweise 4 bis 5 mol. Das Ethylenoxid kann dabei mit einem Inertgas verdünnt werden.

Schließlich wird das so erhaltene Ethylenoxid-Anlagerungsprodukt in wäßriger Lösung zu dem Bis-Betain der Formel I umgesetzt, und zwar in an sich bekannter Weise mit mindestens einem Alkalisalz einer ω-Halogencarbonsäure der Formel $X(CH_2)_{m_1(m_2)}COOH$. Vorzugsweise seien genannt die Alkalisalze und insbesondere die Natriumsalze der Chloressigsäure, Chlorpropionsäure, Bromessigsäure, und der Chlor-n-buttersäure. Gegebenenfalls können Halogencarbonsäure und Alkalihydroxid getrennt zugegeben werden, wobei sich das Salz in situ bildet. Diese Reaktion wird bei einer Temperatur von 80 bis 100 °C mit einem 5 bis 10 %igen Überschuß an Halogencarbonsäure durchgeführt. Vorteilhafterweise stellt man durch geeignete Wahl des Wassergehaltes die erfindungsgemäßen Bis-Betaine der Formel I als 30 bis 40 gew.-%ige wäßrige Einstellungen her.

Die erfindungsgemäßen Bis-Betaine, wie sie in der obengenannten Formel I definiert sind, sind hervorragend geeignet zum Einsatz in kosmetischen Reinigungsmitteln, das heißt Körperreinigungsmitteln wie Schaumbädern, Duschbädern, Fuß- und Handwaschmitteln oder Intimwaschmitteln sowie ferner in Haarwaschmitteln. Beim Einsatz in Körperreinigungsmitteln wird eine deutliche Verbesserung des Hautgefühls nach der Anwendung erzielt, beim Einsatz in Shampoos wird eine Verbesserung der Kämmbarkeit sowohl der trockenen als auch der nassen Haare erreicht bei gleichzeitig weichmachender Wirkung, die sich in einem angenehmen Haargriff bemerkbar macht.

Die erfindungemäßen Bis-Betaine können in solchen flüssigen, pulverförmigen oder auch aerosolförmigen kosmetischen Reinigungsmitteln, insbesondere in Haarwaschmitteln, sowohl allein als auch in Kombination mit den üblicherweise in solchen Mitteln eingesetzten anionischen, kationischen, nichtionischen und amphoteren Tensiden verwendet werden. Dafür geeignete anionische Tenside sind beispielsweise Seife, Fettalkoholsulfate, Alkylethersulfate, Fettsäurekondensationsprodukte wie Tauride, Methyltauride, Sarkoside, ferner α-Olefinsulfonate, Hydroxyalkansulfonate, sekundäre Alkansulfonate, Amidethersulfate oder Alkylbenzolsulfonate. Als nicht-ionische Tenside können beispielsweise Polyglykolmonoalkylether und -monoester, Aminoxide und Ethylenoxid-Propylenoxid-Kondensationsprodukte verwendet werden. Daneben ist auch die Kombination mit anderen amphoteren Tensiden wie Alkyl-Betainen, Alkylamido-Betainen, Imidazolinderivaten oder Sulfo-Betainen möglich. Schließlich können die erfindungsgemäßen Bis-Betaine auch in Abmischung mit kationischen Tensiden wie Cetyltrimethylammoniumchlorid, Cetyltrimethylammoniumbromid, Pentaoxyethylstearylammoniumchlorid, quaternierten Etheraminen oder polymeren quartären Ammoniumverbindungen eingesetzt werden. Weitere Zusätze, die auf sonst übliche Weise in kosmetischen Reinigungsmitteln verwendet werden, können mit den Bis-Betainen kombiniert werden. Dies sind beispielsweise viskositätserhöhende oder -erniedrigende Verbindungen wie Celluloseether, Elektrolyte, wie beispielsweise Natriumchlorid oder Ammoniumchlorid, Fettsäurepolyglykolester, Alkanolamide, Magnesium-Aluminium-Silicate, Polyglykole, Glycerin und Ethanol. Ferner kommen als solche Zusätze in Frage Parfümöle und spezielle Riechstoffe, Antiseptika, schuppenentfernende oder pilztötende Mittel, Überfettungsmittel, Konservierungsmittel, Farbstoffe und Perlglanz gebende Substanzen. Bei der Verarbeitung zu pulverförmigen Präparaten können weiterhin üblicherweise benutzte Füll- und Trägersubstanzen wie hochdisperse, amorphe Kieselsäure, Natriumsulfat, Magnesium-Aluminium-Silicat, Stärkederivate und dergleichen verwendet werden. Schießlich können auch im Falle von Aerosolförmigen Präparaten übliche Treibgase beigemischt werden. Die Regulierung des gewünschten pH-Wertes kann mit anorganischen oder organischen Säuren oder Alkalien vorgenommen werden.

Weiterhin sind die erfindungsgemäßen Bis-Betaine auch zur Formulierung von technischen Reinigungsmitteln, also Schaumreinigern für textile Flächen, wie Teppichreiniger, oder insbesondere Reinigungsmittel für harte Oberflächen, wie beispielsweise Geschirr- und Flaschenspülmittel, Fußbodenreiniger, Sanitärreiniger oder sogenannte Allzweckreinigungsmittel, geeignet. Schließlich eignen sich die erfindungsgemäßen Bis-Betaine als Textilwaschmittel. Auch bei diesen Anwendungsmöglichkeiten können die obengenannten anionischen, kationischen, nicht-ionischen oder amphoteren Tenside beigemischt werden. In technischen Reinigungsmitteln können dabei als übliche Hilfsmittel Chelatbildner und gegebenenfalls auch Kunststoffdispersionen zugesetzt werden. Ferner sind übliche Zusätze hier Bleichmittel, Chlorabspalter oder andere Desinfektionsmittel. Zur Verbesserung der Abrasionswirkung eignen sich Kreide, hochdisperse amorphe Kieselsäure, Phosphate und Kunststoffe. Zur Verbesserung der Fett- und Schmutzlöseeigenschaften können auch Lösungsmittel wie Testbenzin oder Isopropylalkohol oder andere reinigungsverstärkende Mittel zugefügt werden. Schließlich enthalten Waschmittel die üblichen Gerüstsubstanzen.

Ein besonderer anwendungstechnischer Vorteil beim Einsatz der Bis-Betaine in technischen und kosmetischen Reinigungsmitteln ist ihre Stabilität im sauren und alkalischen pH-Bereich. Beispielsweise ist es möglich, lagerstabile Shampoos mit saurem pH-Wert herzustellen, ohne daß — im Gegensatz zu den üblicherweise verwendeten anionischen Alkylsulfaten oder Alkylethersulfaten — Zersetzung durch Hydrolyse eintritt.

Der Gehalt an den erfindungsgemäßen Bis-Betainen in solchen Formulierungen beträgt üblicherweise 0,5 bis 40 Gew.-%.

Die folgenden Beispiele sollen die Erfindung näher erläutern :

## Herstellungsbeispiele

### Beispiel 1

In einem 2-l-Vierhalskolben mit Rückfluß, Thermometer, Rührer und Dosiergefäß werden 670 g Cocosfettamin (Zusammensetzung in Mol-% bezüglich der Reste R : $C_8$ 6 %, $C_{10}$ 6 %, $C_{12}$ 54 %, $C_{14}$ 18 %, $C_{16}$ 8 %, $C_{18}$ 8 %), 68 g Wasser, 34 g Methanol und 14 g konzentrierte Essigsäure auf 60 °C erhitzt. Innerhalb einer Stunde tropft man 373 g Acrylnitril zu und rührt weitere 24 h bei 75 °C unter Rückfluß. Anschließend wird mit 13 g NaOH und 120 g Wasser neutralisiert, das Waschwasser separiert und das Produkt im Vakuum vom Restwasser und Lösungsmittel befreit. Man erhält 1 000 g Cocosfettamino-dipropionitril (Ausbeute 95,9 %).

Ein 5-l-Autoklav wird mit 2 020 g Cocosfettamino-d-propionitril, 3 g Kobalt-Trägerkontakt (Träger : Kieselgur) und 300 ml flüssigen Ammoniaks beschickt. Die Hydrierung erfolgt bei 150 bis 180 bar $H_2$ und 110 bis 140 °C innerhalb von 3 Stunden. Nach Abfiltrieren des Katalysators erhält man 2 010 g Produkt, das 85 bis 95 % Bis(3-aminopropyl)-cocosfettamin enthält.

In einem 2-l-Druckgefäß mit Thermometer, Rührer und Ethylenoxid-Einlaß und -Auslaß werden 954 g Bis(3-aminopropyl)-cocosfettamin auf 130 °C unter Rühren aufgeheizt. 667 g Ethylenoxid werden bei einem Druck von 1 bis 3 bar aufgegeben. Die Gewichtszunahme nach 3 Stunden Reaktionszeit entspricht einem Kondensationsprodukt des Triamins mit 4 bis 5 mol Ethylenoxid. Man erhält 1 605 g dieses Oxethylats (99 %).

244 g dieses Bis(3-aminopropyl)-cocosfettaminoxethylats und 622 g Wasser werden in einem 2-l-Reaktionsgefäß vorgelegt und unter Rühren auf 90 °C erwärmt. Bei dieser Temperatur werden sodann innerhalb von 1 h 113 g Natriumchloracetat zugegeben, anschließend wird noch 12 h bei 95 °C nachgerührt. Man erhält das erfindungsgemäße Bis-Betain in 970 g einer 30 gew.-%igen wäßrigen Lösung.

## Beispiel 2

670 g Laurylamin (Anteil $C_{12}$ 73 Mol-%, Anteil $C_{14}$ 23 Mol-%) werden in 68 g Wasser, 34 g Methanol und 14 g konzentrierter Essigsäure in der bereits in Beispiel 1 beschriebenen Weise mit 373 g Acrylnitril umgesetzt. Nach der Neutralisation und dem Waschen erhält man 1 000 g Laurylamino-di-propionitril.

Nach der Hydrierung von 2 020 g des entsprechenden Dipropionitrils erhält man Bis(3-aminopropyl)-laurylamin in quantitativer Ausbeute. 954 g dieses Triamins werden mit 640 g Ethylenoxid kondensiert. Man erhält 1 590 g an Bis(3-aminopropyl)-laurylaminoxethylat (99 %). 257 g dieses Oxethylats und 676 g Wasser werden mit 116,5 g Natriumchloracetat zur Reaktion gebracht. Man erhält das Bis-Betain in 1 070 g einer 30 %igen wäßrigen Lösung.

## Beispiel 3

844 g Myristylamin werden in 68 g Wasser, 34 g Methanol und 14 g konzentrierter Essigsäure mit 373 g Acrylnitril bei 75 °C umgesetzt. Man erhält 1 164 g Myristylamino-di-propionitril. 2 052 g des Dipropionitrils werden wie in Beispiel 1 mit Kobalt-Katalysator hydriert. Man erhält 2 045 g Bis(3-aminopropyl)-myristylamin. 1 095 g dieses Triamins werden mit 647 g Ethylenoxid kondensiert. Man erhält 1 725 g an Bis(3-aminopropyl)-myristylaminoxethylat (99 %). 290 g dieses Oxethylats und 754 g Wasser werden mit 116,5 g Natriumchloracetat zur Reaktion gebracht. Man erhält das Bis-Betain in 1 160 g einer 30 %igen wäßrigen Lösung.

## Beispiel 4

1 056,0 g Octylamin werden in 106 g Wasser, 53 g Methanol und 21,1 g konzentrierter Essigsäure mit 849,6 g Acrylnitril bei 75 °C umgesetzt. Man erhält 1 810 g Octylamino-di-propionitril. 1 980 g des Di-propionitrils werden wie in Beispiel 1 mit Kobalt-Katalysator hydriert. Man erhält 1 970 g Bis(3-aminopropyl)-octylamin. 998 g dieses Triamins werden mit 845 g Ethylenoxid kondensiert. Man erhält 1 840 g an Bis(3-aminopropyl)-octylaminoxethylat (99 %). 230 g dieses Oxethylats und 613 g Wasser werden mit 116,5 g Natriumchloracetat zur Reaktion gebracht. Man erhält das Bis-Betain in 960 g einer 30 %igen wäßrigen Lösung.

## Beispiel 5

929 g Talgfettamin werden in 68 g Wasser, 34 g Methanol und 14 g konzentrierter Essigsäure mit 373 g Acrylnitril bei 75 °C umgesetzt. Man erhält 1 237 g Talgfettamino-di-propionitril, das gemäß Beispiel 1 zum entsprechenden Amin hydriert wird.

Die Oxethylierungsreaktion wird in zwei Stufen durchgeführt. Zunächst wird das Bis(3-aminopropyl)-talgfettamin nach der Methode von Beispiel 1 mit 4,7 mol Ethylenoxid umgesetzt. Unter Einsatz von üblichen 0,2 Gew.-%, bezogen auf das Amin, an wäßriger Natronlauge (50 %ig) wird anschließend mit weiteren 5,3 mol Ethylenoxid umgesetzt, so daß die gesamte Gewichtszunahme einem Kondensationsprodukt des Triamins mit 10 mol Ethylenoxid entspricht. 300 g dieses Oxethylats und 777 g Wasser werden mit 116,5 g Natriumchloracetat umgesetzt. Man erhält das Bis-Betain in 1 190 g einer 30 %igen wäßrigen Lösung.

Die analytischen Daten der erfindungsgemäßen Bis-Betaine und deren Vorprodukte sind in Tabelle I zusammengefaßt.

(Siehe Tabelle I Seite 6 f.)

Tabelle I

| Beispiel/ (Alkylrest) | Alkylamino-di-propionitril | | Bis(3-aminopropyl)-alkyl-amin | | | | Oxethylat | | | Bis-Betain | |
|---|---|---|---|---|---|---|---|---|---|---|---|
| | AZ | tert. N (%) | AZ | prim. N (%) | sek. N (%) | tert. N (%) | AZ | tert. N (%) | Σ a+b+c+d Äquiv. EO* pro mol | Gesamt-Chlor Gew.-% | ionogenes Chlor Gew.-% |
| 1 (Cocos) | 34,3 | 94 | 97,3 | 66,2 | 3,3 | 30,5 | 57,5 | >98 | 4,9 | 3,4 | 3,3 |
| 2 (Lauryl) | 34,4 | 95 | 97,5 | 65,9 | 2,9 | 31,2 | 58,3 | >98 | 4,7 | 3,4 | 3,3 |
| 3 (Myristyl) | 29,2 | 94 | 82,2 | 65,5 | 3,5 | 31,0 | 51,7 | >98 | 4,9 | 3,3 | 3,2 |
| 4 (Octyl) | 42,7 | 94 | 120,2 | 65,1 | 3,0 | 31,9 | 65,1 | >98 | 4,8 | 4,1 | 4,0 |
| 5 (Talgfett) | 27,4 | 94 | 78,0 | 66,1 | 2,6 | 31,3 | 50,0 | >98 | 4,9 | 3,2 | 3,1 |

*EO = Ethylenoxid

Die obengenannten Daten werden wie folgt bestimmt :

Alkylamino-di-propionitril :

Die Bestimmung der Aminzahl (AZ) und Gehaltes an tertiärem Stickstoff erfolgt durch Titration mit 0,1 N $HClO_4$ in Eisessig bzw. Essigsäureanhydrid. Die Aminzahl errechnet sich aus AZ = (ml 0,1 N $HClO_4$)/Einwaage in g

Bis(3-aminopropyl)-alkylamin :

Die Bestimmung der Aminzahl und der Aminverteilung erfolgt durch Titration mit 0,2 N-isopropanolischer HCl in wasserfreiem Medium. Die Aminverteilung wird durch Blockierung des basischen Aminstickstoffs mit Salicylaldehyd (primäres N) bzw. Phenylisothiocyanat (primäres und sekundäres N) durchgeführt.

Oxethylat :

Die Bestimmung der Aminzahl und des Gehaltes an tertiärem Stickstoff erfolgt durch Titration mit 0,1 N $HClO_4$ in Eisessig bzw. Essigsäureanhydrid.
Die aufgenommenen Mole Ethylenoxid errechnen sich aus den Aminzahlen bzw. aus der Massenzunahme gegenüber der Vorstufe.

Bis-Betain :

Die Bestimmung des Gehaltes an Gesamtchlor erfolgt nach Parr-Aufschluß mit $Na_2O_2$, die Bestimmung des ionogenen Chlors erfolgt durch Titration nach Volhard.
Die nachfolgend aufgeführten Anwendungsbeispiele zeigen die Einsatzmöglichkeiten der Bis-Betaine in Haar- und Körperreinigungsmitteln. Die Mengen- und Prozentangaben in den Beispielen beziehen sich, sofern nicht anders erwähnt, jeweils auf das Gewicht.

### Beispiel 1A

Haarshampoo mit Avivage-Effekt

| | |
|---|---|
| Bis-Betain der Formel I, hergestellt nach Beispiel 1 | 15,00 % |
| Polyethylenglykol-6000-distearat | 5,20 % |
| Parfümöl | 0,30 % |
| Formaldehyd | 0,05 % |
| Wasser | ad 100,00 % |

### Beispiel 2A

Haarshampoo mit avivierendem Effekt

| | |
|---|---|
| Bis-Betain der Formel I, hergestellt nach Beispiel 2 | 12,00 % |
| Hydroxyethylcelluloseether | 1,40 % |
| Parfümöl | 0,30 % |
| Formaldehyd | 0,05 % |
| Wasser | ad 100,00 % |

### Beispiel 3A

Saures Shampoo

| | |
|---|---|
| Bis-Betain der Formel I, hergestellt nach Beispiel 1 | 15,00 % |
| Citronensäure | 0,30 % |
| Parfümöl | 0,10 % |
| Konservierungsmittel, Farbstoffe, Wasser | ad 100,00 % |

### Beispiel 4A

Shampoo für geschädigte Haare

| | |
|---|---|
| Bis-Betain der Formel I, hergestellt nach Beispiel 2 | 10,00 % |
| Lauryltriglykolethersulfat-Natriumsalz | 4,00 % |
| Cocosfettsäurediethanolamid | 2,00 % |
| Natriumchlorid | 3,20 % |

| | |
|---|---|
| Wasser, Konservierungsmittel, Farbstoffe | ad 100,00 % |

Beispiel 5A

Antischuppenshampoo

| | |
|---|---|
| Bis-Betain der Formel I, hergestellt nach Beispiel 2 | 5,00 % |
| Palmkernfettsäuremethyltaurid-Natriumsalz | 6,00 % |
| Stearinsäuremethyltaurid-Natriumsalz | 4,00 % |
| Lauroylsarkosid-Natriumsalz | 2,00 % |
| 2-Mercaptopyridin-N-oxid-Zinksalz | 0,50 % |
| Parfümöl | 0,20 % |
| Wasser, Farbstoffe | ad 100,00 % |

Beispiel 6A

Shampoo für fettige Haare

| | |
|---|---|
| Bis-Betain der Formel I, hergestellt nach Beispiel 2 | 7,00 % |
| sekundäres Alkansulfonat-Natriumsalz (Alkanrest $C_{13}$-$C_{17}$) | 5,00 % |
| $\alpha$-Olefinsulfonat ($C_{14}$-$C_{16}$) | 2,00 % |
| Laurylsulfat-Natriumsalz | 2,00 % |
| Wasser, Konservierungsmittel, Farbstoffe | ad 100,00 % |

Beispiel 7A

Shampoo für trockene Haare

| | |
|---|---|
| Bis-Betain der Formel I, hergestellt nach Beispiel 2 | 10,00 % |
| Lauryldimethylaminoxid | 5,00 % |
| Ethylenglykolmonostearat | 1,20 % |
| Acylaminotriglykolethersulfat-Triethanolaminsalz (Acyl = Capryl- bis Stearinsäurest) | 4,00 % |
| Celluloseether | 1,40 % |
| Wasser, Konservierungsmittel, Farbstoffe | ad 100,00 % |

Beispiel 8A

Duschbad

| | |
|---|---|
| Bis-Betain der Formel I, hergestellt nach Beispiel 2 | 12,00 % |
| Lauryltetraglykolethersulfosuccinat-Dinatriumsalz | 3,00 % |
| Hydroxyethylcelluloseether | 1,20 % |
| Parfümöl | 0,10 % |
| Cocosfettsäuremonoethanolamid | 0,80 % |
| Wasser, Konservierungsmittel, Farbstoffe | ad 100,00 % |

Beispiel 9A

Schaumbad

| | |
|---|---|
| Bis-Betain der Formel I, hergestellt nach Beispiel 2 | 5,00 % |
| Lauryldiglykolethersulfat-Natriumsalz | 20,00 % |
| sekundäres Alkansulfonat-Natriumsalz (Alkanrest $C_{13}$-$C_{17}$) | 5,00 % |
| Cocosfettsäurediethanolamid | 2,00 % |
| Parfümöl | 0,40 % |
| Natriumchlorid | 3,00 % |
| Wasser, Konservierungsmittel, Farbstoffe | ad 100,00 % |

Beispiel 10A

Handwaschmittel

| | |
|---|---|
| Bis-Betain der Formel I, hergestellt nach Beispiel 2 | 10,00 % |
| Laurylsulfat-Natriumsalz | 2,00 % |
| Lauroylsarkosid-Natriumsalz | 4,00 % |

| | |
|---|---|
| Milchsäure | 0,10 % |
| Wasser, Konservierungsmittel, Farbstoffe | ad 100,00 % |

Beispiel 11A

Fußwaschmittel

| | |
|---|---|
| Bis-Betain der Formel I, hergestellt nach Beispiel 1 | 12,00 % |
| Cocosbetain | 2,00 % |
| Cocosdimethylaminoxid | 2,00 % |
| Salicylsäure | 0,50 % |
| Wasser, Parfümöl, Farbstoffe | ad 100,00 % |

Beispiel 12A

Intimwaschmittel

| | |
|---|---|
| Bis-Betain der Formel I, hergestellt nach Beispiel 1 | 7,00 % |
| Cocosethylcycloimidino-1-hydroxy-3-ethylnatriumalkoholat-2-methylnatrium-carboxylat | 7,00 % |
| Lauryltriglykolethersulfat-Triethanolaminsalz | 4,00 % |
| Citronensäure | 0,10 % |
| 3,4,4'-Trichlorcarbanilid | 0,20 % |
| Wasser, Konservierungsmittel, Farbstoffe | ad 100,00 % |

In den folgenden Vergleichsversuchen wird das nach Beispiel 2 hergestellte Bis-Betain üblichen anionischen Tensiden gegenübergestellt. Die Prüfung des Konditionierungseffektes erfolgte sowohl in vitro als auch in vivo nach folgenden Testmethoden :

1. In vitro-Test/Haarkämmbarkeit

15 cm lange mitteleuropäische Haarsträhnen mit einem Durchmesser von 1,5 cm wurden jeweils mit der zu prüfenden 15 % Tensid enthaltenden wäßrigen Lösung gewaschen. Anschließend wurde mit Leitungswasser von + 35 °C 2 Minuten gespült und die Kämmbarkeit des nassen Haares beurteilt. Nach dem Trocknen der Testhaare erfolgte die Prüfung der sogenannten Trockenkämmbarkeit, der Antistatikwirkung und der Naßkämmbarkeit. Die Ergebnisse sind in der Tabelle II zusammengefaßt.

2. In vivo-Test/Halbkopf-Haarkämmbarkeit

Das Kopfhaar von jeweils 5 weiblichen Versuchspersonen wurde in der Mitte gescheitelt und mit 100 ml Leitungswasser von + 35 °C befeuchtet. Jede Kopfhälfte wurde nun mit 5 ml einer 15 %igen tensidhaltigen Lösung gewaschen, wobei das Einschäumen mit synchronen Bewegungen 90 Sekunden lang erfolgte. Anschließend wurde 3 Minuten lang kontinuierlich mit Leitungswasser von + 35 °C ausgespült und die Kämmbarkeit des nassen Haares unter Verwendung von definierten Polyamidkämmen vergleichend geprüft. Der Griff der nassen Haare wurde von neutralen Testpersonen beurteilt. Nach 15 minütiger Trockenzeit wurde ebenfalls von neutralen Testpersonen die Kämmbarkeit des trockenen Haares beurteilt und gleichzeitig der Griff der trockenen Haare und der antistatische Effekt geprüft. Die Ergebnisse sind der Tabelle III zu entnehmen.

Wie die in den Tabellen II und III zusammengefaßten Ergebnisse zeigen, ist es möglich, mit den erfindungsgemäßen Bis-Betainen Haarwaschmittel zu formulieren, die vor allem durch einen außergewöhnlich guten Avivage-Effekt charakterisiert sind ; für den Einsatz in der Praxis bedeutet dies, daß die Haarshampoos für den Verbraucher eine wesentliche Erleichterung bei der Kämmbarkeit der nassen und trockenen Haare bieten und außerdem den Griff der Haare nach der Anwendung positiv beeinflussen.

Beim Einsatz der Bis-Betaine in Körperreinigungsmitteln wurde vor allem eine Verbesserung des Hautgefühls nach der Anwendung gefunden. Die Prüfung erfolgte in vivo nach folgendem Test :

Neutrale Versuchspersonen verstrichen 5 ml einer Mischung aus 50 % Olivenöl und 50 % Paraffinöl auf den Handoberflächen. Anschließend wurden mit 10 ml einer 15 %igen tensidhaltigen wäßrigen Lösung die Hände unter fließendem Leitungswasser von + 25 °C 3 Minuten lang gewaschen. Neben der Beurteilung des Schaumbildes bei dem Reinigungseffekt erfolgte eine Aussage über das Hautgefühl sofort nach dem Abtrocknen und nach 10 Minuten. Die einzelnen Ergebnisse sind in der Tabelle IV zusammengefaßt.

(Siehe Tabellen Seite 10 ff.)

Tabelle II

| Tensid | Naßkämmbarkeit | Trockenkämmbarkeit | Antistatik |
|---|---|---|---|
| Bis-Betain von Beispiel 2 (erfindungsgemäß) | sehr gut | sehr gut | gut |
| Laurylsulfat-Triethanolaminsalz (Vergleich) | schlecht | schlecht | schlecht |
| Lauryldiglykol-ethersulfat-Natriumsalz (Vergleich) | sehr schlecht | mäßig | schlecht |

Tabelle III

| Tensid | Naßkämmbarkeit | Griff der Haare naß | Griff der Haare trocken | Trockenkämmbarkeit | Antistatik |
|---|---|---|---|---|---|
| Bis-Betain von Beispiel 2 (erfindungsgemäß) | sehr gut | glatt, angenehm | weich | sehr gut | sehr gut |
| Laurylsulfat-Triethanolaminsalz (Vergleich) | schlecht | rauh, "bremsend" | spröde | schlecht | schlecht |
| Lauryldiglykol-ether-sulfat-Natriumsalz (Vergleich) | sehr schlecht | schlecht | hart, rauh | mäßig | schlecht |

Tabelle IV

| T e n s i d | Hautgefühl beim Waschen | Schaum-struktur | Hautgefühl sofort nach Abtrocknen | Hautgefühl nach 10 Minuten |
|---|---|---|---|---|
| Bis-Betain von Beispiel 2 (erfindungsgemäß) | angenehm | sahnig, feinblasig | angenehm, geschmeidig | weich, angenehm |
| Laurylsulfat-Natriumsalz (Vergleich) | mäßig | grobblasig, leer | klebrig | sehr trocken, spannt |
| Lauryltriglykol-ether-sulfat-Natriumsalz (Vergleich) | mäßig | grobblasig | klebrig | trocken, spannt |

**Ansprüche** (für die Vertragsstaaten : BE, CH, DE, FR, GB, IT, LI, NL, SE)

1. Bis-Betaine der allgemeinen Formel

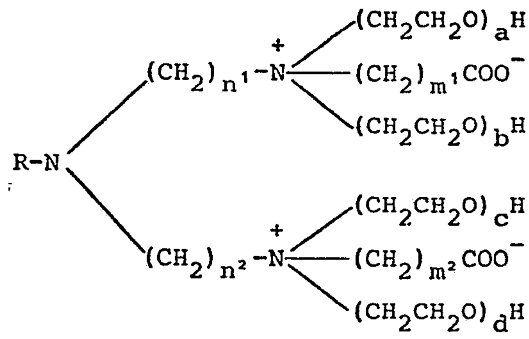

(I)

worin

R einen gesättigten oder einen olefinisch ungesättigten Kohlenwasserstoffrest mit 1 bis 3 Doppelbindungen und von 8 bis 22 C-Atomen bedeutet,

$n^1$ und $n^2$ eine ganze Zahl von 2 bis 3, wobei $n^1$ und $n^2$ gleich oder verschieden sein können,

$m^1$ und $m^2$ eine ganze Zahl von 1 bis 4, wobei $m^1$ und $m^2$ gleich oder verschieden sein können, darstellt, sowie

a, b, c und d, gleich oder verschieden, jeweils eine Zahl von 1 bis 5 ist, wobei die Summe a + b + c + d höchstens 10 betragen soll.

2. Verfahren zur Herstellung von Bis-Betainen gemäß Anspruch 1, bei dem zunächst ein primäres Amin der Formel $RNH_2$ (II) mit 2 mol mindestens eines reaktionsfähigen Nitrils von 2 bis 3 C-Atomen zu einer Verbindung der Formel

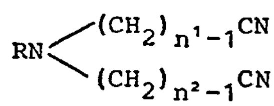

(III)

umgesetzt, in Gegenwart von Wasserstoff zu einer Verbindung der Formel

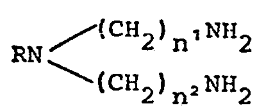

(IV)

reduziert und mit Ethylenoxid zu einer Verbindung der Formel

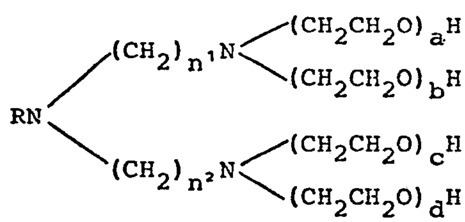

(V)

kondensiert wird, dadurch gekennzeichnet, daß diese Verbindung der Formel (V) in wäßriger Lösung mit mindestens einem Alkalisalz einer ω-Halogencarbonsäure der Formel

$$X(CH_2)_{m_1(m_2)}COOH$$

quaterniert wird.

3. Kosmetisches Reinigungsmittel, enthaltend Wasser als flüssigen Träger, gegebenenfalls mindestens ein Tensid aus der Gruppe der anionischen, kationischen, nicht-ionischen oder amphoteren Tenside, gegebenenfalls übliche kosmetische Zusätze und Hilfsstoffe, gekennzeichnet durch einen Gehalt an einem Bis-Betain der Formel (I) gemäß Anspruch 1.

4. Technisches Reinigungsmittel, enthaltend Wasser als flüssigen Träger, gegebenenfalls mindestens ein Tensid aus der Gruppe der anionischen, kationischen, nicht-ionischen oder amphoteren

Tenside sowie gegebenenfalls reinigungsverstärkende Zusätze und übliche Hilfsstoffe, gekennzeichnet durch einen Gehalt an einem Bis-Betain der Formel (I) gemäß Anspruch 1.

**Ansprüche** (für den Vertragsstaat AT)

1. Verfahren zur Herstellung von Bis-Betainen der allgemeinen Formel

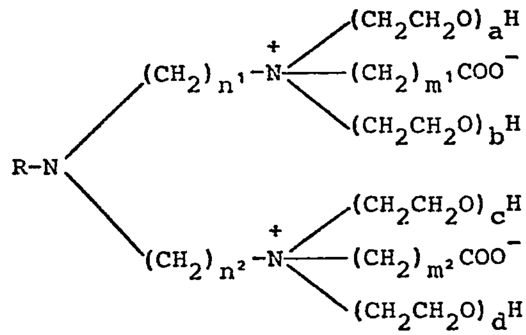

(I)

worin

R einen gesättigten oder einen olefinisch ungesättigten Kohlenwasserstoffrest mit 1 bis 3 Doppelbindungen und von 8 bis 22 C-Atomen bedeutet,

$n^1$ und $n^2$ eine ganze Zahl von 2 bis 3, wobei $n^1$ und $n^2$ gleich oder verschieden sein können,

$m^1$ und $m^2$ eine ganze Zahl von 1 bis 4, wobei $m^1$ und $m^2$ gleich oder verschieden sein können, darstellt, sowie

a, b, c und d, gleich oder verschieden, jeweils eine Zahl von 1 bis 5 ist, wobei die Summe a + b + c + d höchstens 10 betragen soll, bei dem zunächst ein primäres Amin der Formel $RNH_2$ (II) mit 2 mol mindestens eines reaktionsfähigen Nitrils von 2 bis 3 C-Atomen zu einer Verbindung der Formel

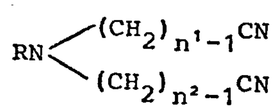

(III)

umgesetzt, in Gegenwart von Wasserstoff zu einer Verbindung der Formel

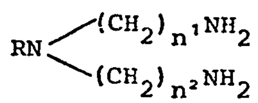

(IV)

reduziert und mit Ethylenoxid zu einer Verbindung der Formel

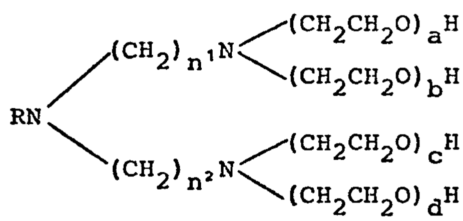

(V)

kondensiert wird, dadurch gekennzeichnet, daß diese Verbindung der Formel (V) in wäßriger Lösung mit mindestens einem Alkalisalz einer ω-Halogencarbonsäure der Formel

$$X(CH_2)_{m_1(m_2)}COOH$$

quaterniert und gegebenenfalls in Substanz isoliert wird.

2. Kosmetisches Reinigungsmittel, enthaltend Wasser als flüssigen Träger, mindestens ein Tensid aus der Gruppe der anionischen, kationischen, nicht-ionischen oder amphoteren Tenside sowie gegebenenfalls übliche kosmetische Zusätze und Hilfsstoffe, gekennzeichnet durch einen Gehalt an einem Bis-Betain der Formel (I) gemäß Anspruch 1.

3. Technisches Reinigungsmittel, enthaltend Wasser als flüssigen Träger, mindestens ein Tensid aus

der Gruppe der anionischen, kationischen, nichtionischen oder amphoteren Tenside sowie gegebenenfalls reinigungsverstärkende Zusätze und übliche Hilfsstoffe, gekennzeichnet durch einen Gehalt an einem Bis-Betain der Formel (I) gemäß Anspruch 1.

**Claims** (for the Contracting States : BE, CH, DE, FR, GB, IT, LI, NL, SE)

1. A bis-betaine of the formula

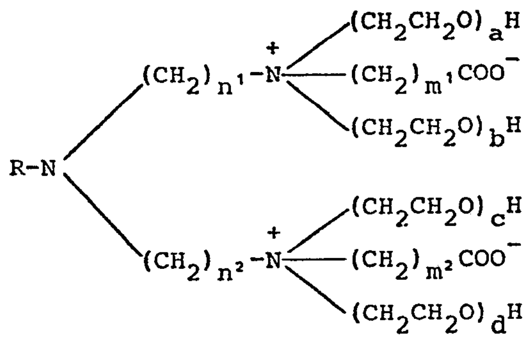

(I)

in which

R denotes a saturated or an olefinically unsaturated hydrocarbon radical which has 1 to 3 double bonds and 8 to 22 carbon atoms,

$n^1$ and $n^2$ represent an integer from 2 to 3, and $n^1$ and $n^2$ can be identical or different,

$m^1$ and $m^2$ represent an integer from 1 to 4, and $m^1$ and $m^2$ can be identical or different, and

a, b, c, and d, which are identical or different, each is a number from 1 to 5, but the sum (a + b + c + d) should be at most 10.

2. A process for preparing a bis-betaine as claimed in claim 1, in which a primary amine of the formula $RNH_2$ (II) is first reacted with 2 moles of at least one reactive nitrile of 2 to 3 carbon atoms to give a compound of the formula

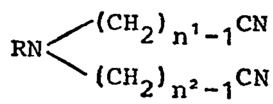

(III)

which is reduced in the presence of hydrogen to give a compound of the formula

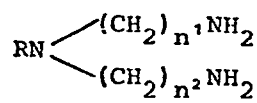

(IV)

which is condensed with ethylene oxide to give a compound of the formula

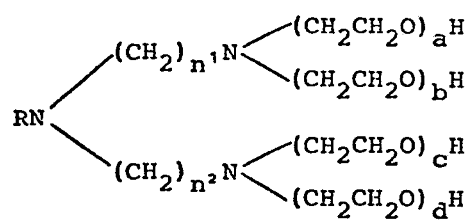

(V)

characterised in that this compound of the formula (V) is quaternised in an aqueous solution with at least one alkali metal salt of an ω-halogenocarboxylic acid of the formula

$$X(CH_2)_{m_1(m_2)}COOH.$$

3. A cosmetic cleaning agent, containing water as a liquid carrier, if appropriate at least one surfactant from the group consisting of anionic, cationic, non-ionic and amphoteric surfactants, if appropriate, customary cosmetic additives and auxiliaries, which is characterised by an amount of a bis-betaine of the formula (I) as claimed in claim 1.

4. An industrial cleaning agent, containing water as a liquid carrier, if appropriate, at least one surfactant from the group consisting of anionic, cationic, non-ionic, and amphoteric surfactants and, if appropriate, cleaning-promoting additives and customary auxiliaries, which is characterised by an amount of a bis-betaine of the formula (I) as claimed in claim 1.

**Claims** (for the Contracting State AT)

1. A process for preparing a bis-betaine of the formula

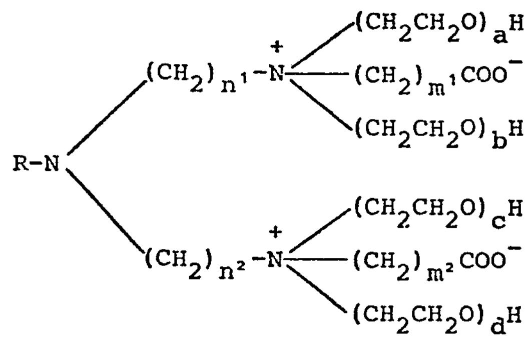

(I)

in which

R denotes a saturated or an olefinically unsaturated hydrocarbon radical which has 1 to 3 double bonds and 8 to 22 carbon atoms,

$n^1$ and $n^2$ represent an integer from 2 to 3, and $n^1$ and $n^2$ can be identical or different,

$m^1$ and $m^2$ represent an integer from 1 to 4, and $m^1$ and $m^2$ can be identical or different, and

a, b, c, und d, which are identical or different, each is a number from 1 to 5, but the sum (a + b + c + d) should be at most 10,

in which a primary amine of the formula $RNH_2$ (II) is first reacted with 2 moles of at least on reactive nitrile of 2 to 3 carbon atoms to give a compound of the formula

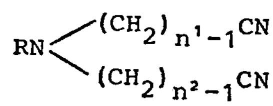

(III)

which is reduced in the presence of hydrogen to give a compound of the formula

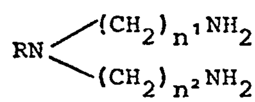

(IV)

which is condensed with ethylene oxide to give a compound of the formula

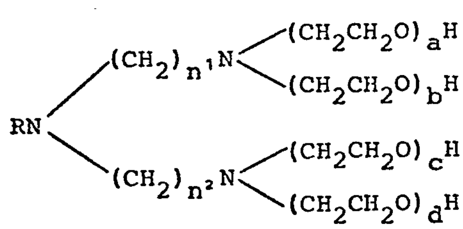

(V)

characterised in that this compound of the formula (V) is quaternised in an aqueous solution with at least one alkali metal salt of an ω-halogenocarboxylic acid of the formula

$$X(CH_2)_{m_1(m_2)}COOH.$$

2. A cosmetic cleaning agent, containing water as a liquid carrier, at least one surfactant from the group consisting of anionic, cationic, non-ionic and amphoteric surfactants, if appropriate, customary cosmetic additives and auxiliaries, which is characterised by an amount of a bis-betaine of the formula (I) as claimed in claim 1.

3. An industrial cleaning agent, containing water as a liquid carrier, at least one surfactant from the group consisting of anionic, cationic, non-ionic, and amphoteric surfactants and, if appropriate, cleaning-promoting additives and customary auxiliaries, which is characterised by an amount of a bis-betaine of the formula (I) as claimed in claim 1.

**Revendications** (pour les Etats contractants : BE, CH, DE, FR, GB, IT, LI, NL, SE)

1. Bis-bétaïnes de formule générale

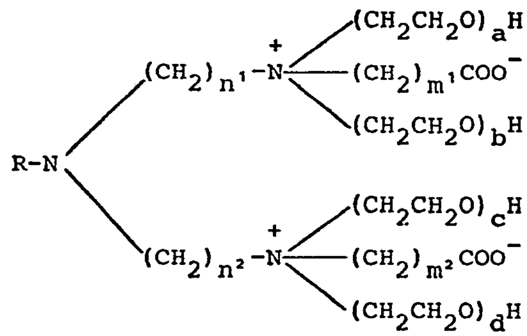

(I)

dans laquelle

R désigne un radical hydrocarboné saturé ou avec de 1 à 3 doubles liaisons oléfiniques, en $C_8$ à $C_{22}$,

$n^1$ et $n^2$, égaux ou différents l'un de l'autre, sont chacun le nombre 2 ou 3,

$m^1$ et $m^2$, égaux ou différents l'un de l'autre, sont des entiers de 1 à 4, et

a, b, c et d, égaux ou différents, sont chacun un nombre de 1 à 5, la somme a + b + c + d ne dépassant pas 10.

2. Procédé de préparation des bis-bétaïnes selon la revendication 1 dans lequel on fait d'abord réagir une amine primaire de formule $RNH_2$ (II) avec deux moles d'un ou de plusieurs nitriles réactifs en $C_2$ ou $C_3$ pour former un composé de formule

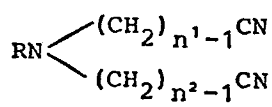

(III)

que l'on réduit par l'hydrogène en un composé de formule

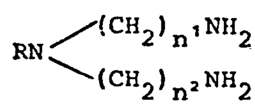

(IV)

composé que l'on condense avec de l'oxyde d'éthylène en un composé de formule

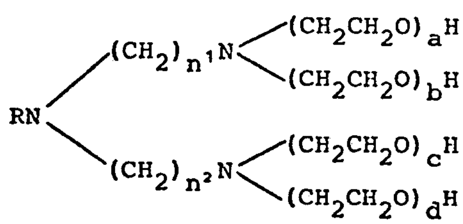

(V)

procédé caractérisé en ce que l'on quaternise ce composé de formule V en solution aqueuse avec un ou plusieurs sels alcalins d'acides ω-halogénocarboxyliques de formule

$$X(CH_2)_{m_1(m_2)}COOH.$$

3. Produits cosmétiques aqueux de lavage contenant éventuellement un ou plusieurs agents tensioactifs choisis parmi des agents anioniques, cationiques, non ioniques et amphotères, avec le cas échéant des additifs et adjuvants courants pour cosmétiques, produits caractérisés en ce qu'ils contiennent une bis-bétaïne de formule I selon la revendication 1.

4. Produits de nettoyage industriels aqueux contenant éventuellement un ou plusieurs agents tensioactifs choisis parmi des agents anioniques, cationiques, non ioniques et amphotères, avec le cas échéant des additifs et adjuvants courants pour renforcer le nettoyage, produits caractérisés en ce qu'ils contiennent une bis-bétaïne de formule I selon la revendication 1.

**Revendications** (pour l'Etat contractant AT)

1. Procédé de préparation de bis-bétaïnes de formule générale

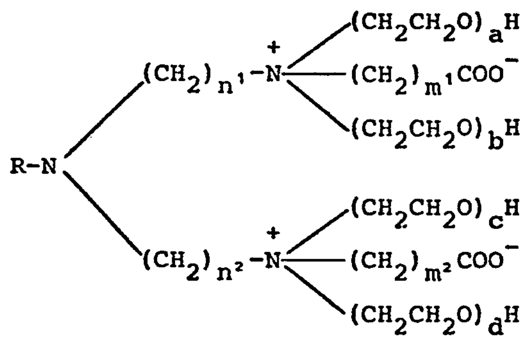

(I)

dans laquelle

R désigne un radical hydrocarboné saturé ou avec 1 à 3 doubles liaisons oléfiniques, en $C_8$ à $C_{22}$,

$n^1$ et $n^2$, égaux ou différents l'un de l'autre, sont chacun le nombre 2 ou 3,

$m^1$ et $m^2$, égaux ou différents l'un de l'autre, sont des entiers de 1 à 4, et

a, b, c et d, égaux ou différents, sont chacun un nombre de 1 à 5, la somme a + b + c + d ne dépassant pas 10, procédé dans lequel on fait d'abord réagir une amine primaire de formule $RNH_2$ (II) avec deux moles d'un ou de plusieurs nitriles réactifs en $C_2$ ou $C_3$ pour former un composé de formule

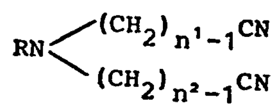

(III)

que l'on réduit par l'hydrogène en un composé de formule

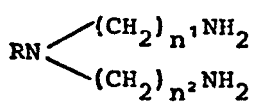

(IV)

composé que l'on condense avec de l'oxyde d'éthylène en un composé de formule

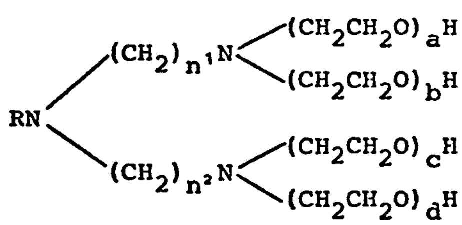

(V)

procédé caractérisé en ce que l'on quaternise ce composé de formule V en solution aqueuse avec un ou plusieurs sels alcalins d'acides ω-halogénocarboxyliques de formule

$$X(CH_2)_{m_1(m_2)}COOH$$

et le cas échéant on isole la substance formée.

2. Produits cosmétiques aqueux de lavage contenant éventuellement un ou plusieurs agents tensioactifs choisis parmi des agents anioniques, cationiques, non ioniques et amphotères, avec le cas échéant des additifs et adjuvants courants pour cosmétiques, produits caractérisés en ce qu'ils contiennent une bis-bétaïne de formule I selon la revendication 1.

3. Produits de nettoyage industriels aqueux contenant éventuellement un ou plusieurs agents tensioactifs choisis parmi des agents anioniques, cationiques, non ioniques et amphotères, avec le cas échéant des additifs et adjuvants courants pour renforcer le nettoyage, produits caractérisés en ce qu'ils contiennent une bis-bétaïne de formule I selon la revendication 1.